# EUROPÄISCHE PATENTANMELDUNG

(11) **EP 4 427 726 A1**
(43) Veröffentlichungstag der Anmeldung: **11.09.2024**
(21) Anmeldenummer: 23160281.4
(22) Anmeldetag: 06.03.2023
(51) Int. Cl.: A61K 8/04, A61K 8/34, A61K 8/92, A61K 8/97, A61Q 17/00, A61Q 19/00

(54) **KOSMETISCHE ZUSAMMENSETZUNG AUFWEISEND EIN 2-PHASEN GEMISCH**

(71) Anmelder: Weleda AG, 4144 Arlesheim (CH)
(72) Erfinder: MATZKE, Benjamin, 68220 Leymen (FR); POST, Katharina, 4055 Basel (CH)
(74) Vertreter: Simandi, Claus

(57) **Zusammenfassung**

Die Erfindung betrifft eine kosmetische Zusammensetzung, welche zwei Phasen aufweist, jedoch beim Mischen eine homogene stabile Phase erreicht, und vorzugsweise über ein Spray auf die Haut aufgetragen werden kann.

## Beschreibung

Die Erfindung betrifft eine kosmetische Zusammensetzung, welche zwei Phasen aufweist, jedoch beim Mischen eine homogene stabile Phase erreicht, und vorzugsweise über ein Spray auf die Haut aufgetragen werden kann.

Weleda AG forscht mit modernen und in Verbindung mit anthroposophischen Methoden an vorteilhaften natürlichen Pflanzenstoffen für die Kosmetik.

Die Haut, insbesondere die Epidermis, ist als Barriereorgan des menschlichen Organismus in besonderem Maße äußeren Einwirkungen unterworfen (Kälte, Chemikalien, Stress, Umwelteinflüsse, etc.). Diese Barrierefunktion wird unter anderem von Hautlipiden aufrechterhalten. Diese epidermalen Lipide wie z.B. Glycosphingolipide, Ceramide, Sterine und Sterinester, Fettsäuren, Triglyceride, n-Alkane oder verschiedene polare Lipide werden im Keratinisierungsprozess freigesetzt.

In einem optimalen Zustand der Haut liegt ein ausgewogenes Verhältnis von Hautlipiden und Hautfeuchtigkeit vor. Durch dieses Gleichgewicht werden wichtige Eigenschaften der Haut, wie das Penetrationsvermögen, Wasserbindungsvermögen, Elastizität, Regenerationsfähigkeit oder die Widerstandsfähigkeit gegen Umwelteinflüsse und Noxen unterschiedlichster Art, bestimmt. Pflanzenwirkstoffe, insbesondere Sekundärmetabolite, können einen positiven Einfluss auf die Haut nehmen. Es bedarf jedoch schonender Mittel zur Pflege und Reinigung der Haut.

Der vorliegenden Erfindung liegt daher die Aufgabe zugrunde, eine kosmetische Zusammensetzung zur topischen Verwendung bereitzustellen, sodass wertvolle Pflanzeninhaltsstoffe vorteilhaft in die Haut penetrieren können und ein zusätzlicher Pflegeeffekt erreicht wird. Zumeist liegen solche natürliche Pflanzeninhaltsstoffe als sekundäre Pflanzenmetabolite vor, welche sowohl wasserlöslich als auch fettlöslich sein können.

Überraschender Weise konnten die Erfinder feststellen, dass solche natürlichen Pflanzeninhaltsstoffe, insbesondere Pflanzeninhaltsstoffe als sekundäre Pflanzenmetabolite, in einer 2-Phasenmischung nach Mischen eine homogene Phase ausbilden und besonders vorteilhaft topisch auf die Haut aufgebracht werden können, und auf diese Weise vorteilhaft Pflanzeninhaltsstoffe in die Haut penetrieren können.

Die Aufgabe wird daher durch die technische Lehre von mindestens einem Patentanspruch gelöst.

Insbesondere betrifft die Erfindung eine kosmetische Zusammensetzung enthaltend ein 2 Phasen Gemisch mit
a.) mehr als 60 Gew. % einer wässrigen Phase, aufweisend mindestens ein Salz mit einem Gehalt von 0,25 - 5 Gew. % in der wässrigen Phase,
b.) weniger als 40 Gew. % einer öligen Phase, aufweisend mindestens ein Pflanzenöl und Fettsäureester im Verhältnis von 1:1 bis 1:3 (v/v) mit einem Gehalt von mindestens 80 Gew. % in der öligen Phase,
c.) wobei mindestens ein lipophiler Auszug und / oder hydrophiler Auszug eines Pflanzenextrakts aufweisend ein oder mehrere Pflanzeninhaltsstoffe mit mindestens einem sekundären Pflanzenstoff in a.) und / oder b.) enthalten ist oder sind, und der Gehalt jeweils in der wässrigen Phase oder öligen Phase maximal 10 Gew. % beträgt,
und ggfs. weiteren Hilfs- und Zusatzstoffen (nachstehend: erfindungsgemäße Zusammensetzung).

Gewichtsangaben beziehen sich auf 100 % einer erfindungsgemäßen kosmetischen Zusammensetzung in totaler Zusammensetzung oder sie beziehen sich auf die jeweilige Phase a.) oder b.).

Eine 100 %-ige Ausführungsform kann dem Beispiel 1 entnommen werden. In Beispiel 2 wird in wässrige Phase und ölige Phase unterschieden.

Die vorgenannte kosmetische Zusammensetzung kann durch Zuführung von Bewegungsenergie, wie Mischen, insbesondere Schütteln oder Rühren in eine homogene Phase überführt werden, wobei die zwei Phasen sich vermengen. Die homogene Phase ist über mehrere Minuten oder gar Stunden haltbar oder stabil. Sobald eine solche Homogenität zumindest teilweise erreicht wird, kann eine Auftragung auf die Haut erfolgen.

Daher betrifft die Erfindung eine erfindungsgemäße kosmetische Zusammensetzung, wobei durch Mischen vorteilhaft eine homogene Phase erhalten wird, und die lipo- und hydrophilen Pflanzeninhaltsstoffe mit mindestens einem sekundären Pflanzenstoff gelöst sind, die topisch auf die Haut aufgetragen werden können.

Daher betrifft die Erfindung eine erfindungsgemäße kosmetische Zusammensetzung, wobei durch Mischen eine homogene Phase erhalten wird, welche auf die Haut aufgetragen werden kann.

Besonders vorteilhaft kann die erfindungsgemäße Zusammensetzung nach Schütteln mithilfe eines Zerstäubers oder Sprays auf die Haut verteilt werden. Die Flasche kann zumindest teilweise transparent sein, sodass das Erhalten der homogenen Phase nach Schütteln mit dem Auge sichtbar ist.

In einer weiteren Ausführungsform der Erfindung ist bevorzugt, dass die wässrige Phase ein lösliches Salz aufweist, vorzugsweise Natriumchlorid oder Meersalz mit einem Gehalt von 0,25 - 5 Gew. % in der wässrigen Phase, insbesondere 0,5 - 2,5 Gew. %., besonders bevorzugt 1,5 Gew. %.

In einer weiteren Ausführungsform der Erfindung ist bevorzugt, dass das Verhältnis von Pflanzenöl und Fettsäureestern in der öligen Phase 1:1 bis 1:3 (v/v), insbesondere 1:1 bis 1:1,5 und 1: 1,5 bis 2: 2,5 (v/v) beträgt mit einem Gehalt von mindestens 80 Gew. % in der öligen Phase, insbesondere von mindestens 90 Gew. % in der öligen Phase, besonders bevorzugt 95 - 98 Gew. %.

Die erfindungsgemäßen vorgenannten Phasen sind wesentlich für eine geeignete Dichtedifferenz der Phasen, sodass sich zwei Phasen ausbilden. Auf diese Weise wird ein Dichtegradient hergestellt, der die Phasen voneinander trennt. Weiterhin stellt das Salz als Elektrolyt in der wässrigen Phase eine hinreichende Polarität her.

Weiterhin kann die wässrige Phase mindestens einen Alkohol enthalten, vorzugsweise Ethanol oder Glycerin. Der Gehalt an Alkohol beträgt 1 - 30 Gew. %, insbesondere 10 - 25 Gew. %. Bei Zwei-, drei oder vierwertigen Alkoholen sind solche ausgewählt aus der Gruppe Ethandiol, Glycerin, Propandiol, Erythrit, Butandiol, Pentandiol, C1-C5 Alkohole mit 2, 3, 4 oder 5 OH-Gruppen. Glycerin ist jedoch erfindungsgemäß bevorzugt.

In einer weiteren Ausführungsform der Erfindung ist bevorzugt, dass das Pflanzenöl in der öligen Phase b.) mindestens zu 35 Gew. %, insbesondere mindestens zu 40 Gew. % enthalten ist.

Pflanzenöl im Sinne dieser Erfindung bedeutet, dass ein Trioder Diglycerid vorliegt, wobei mindestens eine Fettsäure einoder mehrfach ungesättigt ist, insbesondere solche Fettsäuren mit 14 bis 26 C-Atomen, insbesondere Ölsäure, Linolsäure oder Linolensäure. Das Pflanzenöl ist bei Raumtemperatur flüssig. Solche nativen Pflanzenöle können weitere Substituenten enthalten, wie OH, NH₂ u.a.. Solche Pflanzenöle werden durch verschiedene Verfahren gewonnen, wie Pressen (Kaltpressen, Heißpressen), Extraktion mit Lösungsmitteln (Heptan, Petrolether; u. a.), Superfluid-Chromatographie, o.a..

Ein oder mehrere Pflanzenöle im Sinne dieser Erfindung können aus der Gruppe ausgewählt sein, wie nicht abschließend Açaíöl, Algenöl, Arganöl Avocadoöl, Babaçuöl, Borretschöl, , Distelöl, Erdnussöl, Haselnussöl, Hanföl, Jatrophaöl, Jojobaöl, Kakaobutter, Kokosöl, Kürbiskernöl, Leinöl, Macadamiaöl, Maiskeimöl, Mandelöl, Marillenkernöl, Nachtkerzenöl, Olivenöl, Palmöl, Palmkernöl, Papayaöl, Pistazienöl, Pekannussöl, Perillaöl, Rapsöl, Reisöl, Rizinusöl, Senföl, Sesamöl, Sheabutter, Sojaöl, Sonnenblumenöl, Tungöl, Walnussöl, Wassermelonensamenöl, Traubenkernöl.

Fettsäureester im Sinne dieser Erfindung bedeutet, dass eine Fettsäure mit einem Alkohol verestert ist, und zwar vorzugsweise in Form der mittelkettigen Triglyceride (MKT) bzw. MCT-Fette, also Triglyceride, die mittelkettige Fettsäuren enthalten. Zu den mittelkettigen Fettsäuren zählen die Capron- (C 6:0), Capryl- (C 8:0), Caprin- (C 10:0) und die Laurinsäure (C 12:0). Sog. Caprylic-/ Capric Triglyceride sind käuflich erwerblich. Andere Fettsäureester sind nicht ausgeschlossen, insbesondere solche mit C12 bis C26-Fettsäuren, wobei der Alkohol Methanol oder Ethanol ist.

Ein lipophiler Auszug und / oder hydrophiler Auszug eines Pflanzenextrakts mit Pflanzeninhaltsstoffen sind erfindungsgemäß solche, die sekundäre Pflanzenstoffe enthalten oder aus diesen bestehen. Sekundäre Pflanzenstoffe, bzw. Sekundärmetaboliten leiten sich von Produkten des anabolen und katabolen Stoffwechsels ab, insbesondere von Kohlenhydraten und Aminosäuren. Erfindungsgemäße sekundäre Pflanzenstoffe umfassen insbesondere phenolische, isoprenoide oder alkaloide Verbindungen, wie Phenole, Polyphenole, Flavonoide, Kaffeesäurederivate, Xanthone, Terpene, Steroide u.a. Naturstoffe. Solche Pflanzeninhaltsstoffe können wässrige und / oder ethanolische, oder ölige Auszüge / Extrakte aus einem Pflanzenmaterial sein, insbesondere aus Blättern, Wurzeln, Samen, Blüten. Beispielsweise können solche sekundären Pflanzenstoffe in ätherischen Pflanzenölen vorliegen (z.B. Terpene, etc.). Solche Pflanzenmaterialien können ausgewählt sein aus der Gruppe der Pflanzengattungen Aloe, Althaea, Angelica, Arnika, Artemisia, Calendula, Cannabis, Capsicum, Carum, Citrus, Centella, Echinacea, Hedeara, Humulus, Iberis, Iris, Juglans, Lavendula, Lepidium, Matricaria, Melissa, Mentha, Basilicum (Ocimum), Passiflora, Pelargonium, Primula, Punica, Quercus, Rosmarinus, Rumex, Salix, Salvia, Sambucus, Strychnos, Thymus, Vaccinium, Valeriana, Vebena, Viola, Vitex, Vitis.

In einer weiteren Ausführungsform der Erfindung ist bevorzugt, dass mindestens ein lipophiler Auszug und / oder hydrophiler Auszug eines Pflanzenextrakts aufweisend ein oder mehrere Pflanzeninhaltsstoffe mit mindestens einem sekundären Pflanzenstoff in Phase a.) und / oder Phase b.) enthalten ist oder sind, und der Gehalt jeweils in der wässrigen Phase oder öligen Phase maximal 10 Gew. %, oder mehr als 0,1 Gew. %, insbesondere mehr als 1 Gew. %, maximal 5 Gew. % beträgt.

Die erfindungsgemäßen kosmetischen Zusammensetzungen können weiterhin kosmetische Hilfsstoffe enthalten, wie sie üblicherweise in solchen Zubereitungen verwendet werden, z. B. Antioxidantien, Radikalfänger, Konservierungsmittel, Bakterizide, Parfüme, Farbstoffe, Pigmente, die eine färbende Wirkung haben, Verdickungsmittel, oberflächenaktive Substanzen, weichmachende, oder andere übliche Bestandteile einer kosmetischen Formulierung wie Schaumstabilisatoren, Elektrolyte, u.a.

Die erfindungsgemäßen kosmetischen Zusammensetzungen können insbesondere mindestens ein Feuchtigkeitsmittel enthalten, insbesondere Betaine mit einem Gehalt von 1 - 6 Gew. %, insbesondere zu 2 - 4 Gew. %.

Nachfolgende Ausführungsbeispiele und Figuren dienen zur Erläuterung der Erfindung, ohne die Erfindung auf diese Beispiele zu beschränken.

### Beispiel 1:

Beispielhafte Zusammensetzung einer erfindungsgemäßen Zusammensetzung (Totalzusammensetzung):

| **Inhaltsstoff** | **Funktion** | **Gewicht (Gew. %)** |
|---|---|---|
| Wasser, gereinigt (Bulk) | | **45 Gew. %** |
| Glycerin | | **7 Gew. %** |
| Ethanol (96 %-ig) | | **7 Gew. %** |
| Betaine | Feichtigkeitsmittel | **3 Gew %** |
| Meersalz, NaCl | | **1,5 Gew. %** |
| Sonnenblumenöl | Pflanzenöl | **14,4 Gew. %** |
| Jojobaöl | Pflanzenöl | **3 Gew. %** |
| MCT-Fette (Caprylic-/ Capric Triglyceride) | Fettsäureester | **15 Gew. %** |
| Viola, Herba Extrakt | hydrophiler Auszug eines | **1 Gew. %** |
| | Pflanzenextrakts | |
| Rosmarinöl, Kamillenöl, Calendulaöl, | lipophiler Auszug eines | **2 Gew. %** |
| | Pflanzenextrakts | |
| Parfum | Duftstoff | **0,7 Gew. %** |
| Zusatzstoff(e) | Xanthan, Tocopherol,o.a. | **0,4 Gew. %** |
| | | **100 Gew. %** |

### Beispiel 2:

### a.) Beispielhafte Zusammensetzung einer erfindungsgemäßen Zusammensetzung der wässrigen Phase:

| **Inhaltsstoff** | **Funktion** | **Gewicht (Gew. %)** |
|---|---|---|
| Wasser, gereinigt (Bulk) | | **69,7 Gew. %** |
| Glycerin | | **11 Gew. %** |
| Ethanol (96 %-ig) | | **11 Gew. %** |
| Betaine | | **4,6 Gew. %** |
| Viola, Herba Extrakt | hydrophiler Auszug eines | **1,5 Gew. %** |
| | Pflanzenextrakts | |
| Meersalz, NaCl | | **2,3 Gew. %** |
| Zusatzstoff(e) | Xanthan, o.a. | **0,2 Gew. %** |
| | | **100 Gew. %** |

### b.) Beispielhafte Zusammensetzung einer erfindungsgemäßen Zusammensetzung der öligen Phase:

| **Inhaltsstoff** | **Funktion** | **Gewicht (Gew. %)** |
|---|---|---|
| Sonnenblumenöl | Pflanzenöl | **40,7 Gew. %** |
| Jojobaöl | Pflanzenöl | **8,5 Gew. %** |
| MCT-Fette (Caprylic-/ Capric Triglyceride) | Fettsäureester | **42,4 Gew. %** |
| Rosmarinöl, Kamillenöl, Calendulaöl, | lipophiler Auszug eines | **5,6 Gew %** |
| | Pflanzenextrakts | |
| Parfum | Duftstoff | **2,0 Gew %** |
| Zusatzstoff(e) | Xanthan, Tocopherol,o.a. | **0,8 Gew %** |
| | | **100 Gew %** |

### Beispiel 3:

### Herstellung einer erfindungsgemäßen Zusammensetzung:

Wässrige Phase 1: Wasser, Glycerin, wasserlöslicher Extrakt (Pflanzeninhaltsstoffe) und Meersalz gut vermischen, dann Feststoffe und etwaige Zusatzstoffe vormischen und in die Phase 1 eingeben, rühren, bis eine homogene Mischung erhalten wird.

Ölige Phase 2: Pflanzenöle, Fettsäureester und öllösliche Extrakte (Pflanzeninhaltsstoffe) homogen vermischen, ggf. Parfüm und etwaige Zusatzstoffe hinzufügen.

Anschließend wird Phase 1 und Phase 2 in die (Spray-)Flasche gefüllt.

## Patentansprüche

1. Kosmetische Zusammensetzung enthaltend ein 2 Phasen Gemisch mit
a.) mehr als 60 Gew. % einer wässrigen Phase, aufweisend mindestens ein Salz mit einem Gehalt von 0,25 - 5 Gew. % in der wässrigen Phase,
b.) weniger als 40 Gew. % einer öligen Phase, aufweisend mindestens ein Pflanzenöl und Fettsäureester im Verhältnis von 1:1 bis 1:3 (v/v) mit einem Gehalt von mindestens 80 Gew. % in der öligen Phase,
c.) wobei mindestens ein lipophiler Auszug und / oder hydrophiler Auszug eines Pflanzenextrakts aufweisend ein oder mehrere Pflanzeninhaltsstoffe mit mindestens einem sekundären Pflanzenstoff in a.) und / oder b.) enthalten ist oder sind, und der Gehalt jeweils in der wässrigen Phase oder öligen Phase maximal 10 Gew. % beträgt,
und ggfs. weiteren Hilfs- und Zusatzstoffen.

2. Kosmetische Zusammensetzung nach Anspruch 1, wobei durch Mischen eine homogene Phase erhalten wird, welche auf die Haut aufgetragen werden kann.

3. Kosmetische Zusammensetzung nach Anspruch 1 oder 2, wobei die wässrige Phase a.) Alkohol enthält, und ausgewählt ist aus der Gruppe Ethanol, Ethandiol, Glycerin, Propandiol, Erythrit, Butandiol, Pentandiol, C1-C5 Alkohole mit 2, 3, 4 oder 5 OH-Gruppen.

4. Kosmetische Zusammensetzung nach einem der vorstehenden Patentansprüche, wobei das Pflanzenöl gemäß b.) ausgewählt ist aus der Gruppe Açaíöl, Algenöl, Arganöl Avocadoöl, Babaçuöl, Borretschöl, Distelöl, Erdnussöl, Haselnussöl, Hanföl, Jatrophaöl, Jojobaöl, Kakaobutter, Kokosöl, Kürbiskernöl, Leinöl, Macadamiaöl, Maiskeimöl, Mandelöl, Marillenkernöl, Nachtkerzenöl, Olivenöl, Palmöl, Palmkernöl, Papayaöl, Pistazienöl, Pekannussöl, Perillaöl, Rapsöl, Reisöl, Rizinusöl, Senföl, Sesamöl, Sheabutter, Sojaöl, Sonnenblumenöl, Tungöl, Walnussöl, Wassermelonensamenöl, Traubenkernöl.

5. Kosmetische Zusammensetzung nach einem der vorstehenden Patentansprüche, wobei der Fettsäurester gemäß b.) ein MCT-Fett oder Triglycerid ist.

6. Kosmetische Zusammensetzung nach einem der vorstehenden Patentansprüche, wobei das Verhältnis Pflanzenöl und Fettsäureester gemäß b.) 1 : 1,5 bis 1,5 : 2 (v/v) beträgt.

7. Kosmetische Zusammensetzung nach einem der vorstehenden Patentansprüche, wobei das Pflanzenöl gemäß b.) mindestens zu 35 Gew. %, insbesondere mindestens zu 40 Gew. % enthalten ist.

8. Kosmetische Zusammensetzung nach einem der vorstehenden Patentansprüche enthaltend mindestens ein oder mehrere Pflanzeninhaltsstoffe gemäß Anspruch 1 c.), wobei mindestens ein sekundärer Pflanzenstoff vorliegt, ausgewählt aus der Gruppe phenolische, isoprenoide oder alkaloide Verbindungen, Phenole, Polyphenole, Flavonoide, Kaffeesäurederivate, Xanthone, Terpene, Steroide oder aus wässrigen und / oder ethanolischen, oder öligen Auszügen / Extrakte aus einem Pflanzenmaterial.

9. Kosmetische Zusammensetzung nach Anspruch 8, wobei der Pflanzenextrakt ausgewählt ist aus der Gruppe Aloe, Althaea, Angelica, Arnika, Artemisia, Calendula, Cannabis, Capsicum, Carum, Citrus, Centella, Echinacea, Hedeara, Humulus, Iberis, Iris, Juglans, Lavendula, Lepidium, Matricaria, Melissa, Mentha, Basilicum (Ocimum), Passiflora, Pelargonium, Primula, Punica, Quercus, Rosmarinus, Rumex, Salix, Salvia, Sambucus, Strychnos, Thymus, Vaccinium, Valeriana, Vebena, Viola, Vitex, Vitis.

10. Kosmetische Zusammensetzung nach einem der vorstehenden Patentansprüche, wobei mindestens ein lipophiler Auszug und / oder hydrophiler Auszug eines Pflanzenextrakts aufweisend ein oder mehrere Pflanzeninhaltsstoffe mit mindestens einem sekundären Pflanzenstoff in a.) und / oder b.) enthalten ist oder sind, und der Gehalt jeweils in der wässrigen Phase oder öligen Phase maximal 5 Gew. % beträgt.

11. Kosmetische Zusammensetzung nach einem der vorstehenden Patentansprüche aufweisend
in der wässrigen Phase:
| **Inhaltsstoff** | **Gewicht (Gew. %)** |
|---|---|
| Wasser, gereinigt (Bulk) | **69,7 Gew. %** |
| Glycerin | **11 Gew. %** |
| Ethanol (96 %-ig) | **11 Gew. %** |
| Betaine | **4,6 Gew. %** |
| Viola, Herba Extrakt | **1,5 Gew. %** |
| Meersalz, NaCl | **2,3 Gew. %** |
| Zusatzstoff(e) | **0,2 Gew. %** |
in der öligen Phase:
| **Inhaltsstoff** | **Gewicht (Gew. %)** |
|---|---|
| Sonnenblumenöl | **40,7 Gew. %** |
| Jojobaöl | **8,5 Gew. %** |
| MCT-Fette (Caprylic-/ Capric Triglyceride) | **42,4 Gew. %** |
| Rosmarinöl, Kamillenöl, Calendulaöl, | **5,6 Gew %** |
| Parfum | **2,0 Gew %** |
| Zusatzstoff(e) | **0,8 Gew %** |

12. Spray oder Zerstäuber enthaltend eine kosmetische Zusammensetzung nach einem der vorhergehenden Ansprüche.
